# EUROPEAN PATENT SPECIFICATION

(11) **EP 1 819 320 B1**
(45) Date of publication and mention of the grant of the patent: **01.10.2008**
(21) Application number: 05817592.8
(22) Date of filing: 25.11.2005
(51) Int. Cl.: A61K 9/20

(54) **ORALLY DISPERSIBLE PHARMACEUTICAL COMPOSITION AND PROCESS FOR THE PREPARATION THEREOF**
ORAL DISPERGIERBARE PHARMAZEUTISCHE ZUSAMMENSETZUNG UND VERFAHREN ZU IHRER HERSTELLUNG
COMPOSITION PHARMACEUTIQUE ORODISPERSIBLE ET PROCEDE DE PREPARATION DE CELLE-CI

(30) Priority: 10.12.2004 IT MI20042356
(43) Date of publication of application: 22.08.2007
(73) Proprietor: Aziende Chimiche Riunite Angelini Francesco A.C.R.A.F. S.p.A., 00181 Roma (IT)
(72) Inventor: MARCHITTO, Leonardo, I-62017 Porto Recanati (IT); RAGNI, Lorella, I-60033 Chiaravalle (IT); DONATI, Luca, I-63017 Porto San Giorgio (IT); VALENTI, Mauro, I-20013 Magenta (IT)
(74) Representative: Marchi, Massimo
(86) International application number: PCT/EP2005/012859
(87) International publication number: WO 2006/061142

(56) References cited:
- EP-A- 1 166 777
- WO-A-99/44580

## Description

### FIELD OF THE INVENTION

This invention relates to an orally dispersible pharmaceutical composition comprising an active ingredient coated with at least one hydrophilic carboxylate polymer and at least one lipid compound, wherein said coated active ingredient is embedded in a matrix comprising at least one hydrophilic natural polymer having high molecular weight, and also relates to a process for the preparation thereof.

### STATE OF THE ART

Many techniques relating to the masking of taste or the release of active ingredients of unpleasant taste are described in the art. The most significant examples are listed below.

US patent 4, 916,161 describes a wet granulation process for masking the taste of ibuprofen or other active ingredients having an unpleasant taste using hydroxypropyl methylcellulose phthalate. This document does not however mention the microencapsulation technique.

US patent 4,946,648 describes a pharmaceutical form which will disaggregate rapidly in water, obtained by lyophilisation and comprising a mixture of mannitol and at least one natural gum.

US patent 5,084,278 describes a pharmaceutical formulation of an active ingredient microencapsulated in microcapsules consisting of ethyl cellulose in combination with methacrylic acid ester copolymers or styrene acrylate copolymers. The process of preparation is carried on in a fluid bed drier.

US patents 5,215,755 and US 5,320,855 describe the preparation of coated tablets by means of the rotary granulation technique. The granulation mixture comprises the active ingredient, for example ibuprofen, and excipients such as, for example, polyvinyl pyrrolidone, sodium starch glycolate and sodium lauryl sulphate. The coating material comprises hydroxyethylcellulose or a mixture of hydroxyethylcellulose and hydroxypropylmethylcellulose.

US patent 5,298,261 describes the preparation of a readily disaggregable tablet using a lyophilisation process. The preferred excipients are a gum such as, for example, arabic gum, guar gum, xanthorea resin, carrageenan gum or tragacanth gum, and carbohydrates such as, for example, mannitol, dextrose, sucrose, lactose, maltose, maltodextrin or maize syrup.

US patent 5,405,617 describes a pharmaceutical formulation obtained by incorporating the active ingredient in a mixture of fatty acid esters sprayed in the molten state in motion in a fluid bed coating device.

US patent 5,460,825 describes the preparation of chewable tablets by means of compression of granules obtained by the rotary granulation technique and coated with cellulose acetate, cellulose acetate butyrate, or combinations thereof and hydroxypropylcellulose. The granulation mixture comprises the active ingredient, for example famotidine, binders such as, for example, hydroxypropylmethyl cellulose, and vehicles such as, for example, lactose.

US patent 5,466,464 describes a solid preparation which dissolves in the mouth comprising the active ingredient, a carbohydrate such as, for example, lactose and/or mannitol, and agar. The sugar matrix containing the active ingredient is obtained by dissolution and subsequent drying. The obtained solid preparations have a greater hardness than similar formulations and can therefore be easily removed from blisters.

US patent 5,489,436 describes the preparation of chewable tablets obtained from an active ingredient coated by means of the fluid bed technique with a mixture of dimethylaminoethyl methacrylate and methacrylic acid ester and a cellulose ester such as, for example, cellulose acetate, cellulose acetate butyrate, cellulose triacetate or mixtures thereof, and, optionally, polyvinyl pyrrolidone.

US patent 5,464,632 describes the preparation of tablets containing the active ingredient in the form of coated microcrystals or coated or uncoated microgranules in combination with a disaggregating agent (carboxymethylcellulose or cross-linked PVP) and a swelling agent (amides, modified amides, microcrystalline cellulose) and a sugar, by direct compression.

US patent 5,501,861 describes the preparation of a readily dispersible tablet by means of wet granulation of the active ingredient in a mixture with carbohydrates such as, for example, sugar, starch sugars, lactose, honey, alcohol derivatives of sugars and tetroses, with a small quantity of water.

US patent 5,576,014 describes the preparation of tablets which are soluble in the mouth by means of fluid bed granulation of a mixture of the active ingredient and saccharides having low and high plasmability, and subsequent compression of the granulate.

US patent 5,728,403 describes a method for masking taste by coating particles of active ingredient with a mixture of a triglyceride and a copolymer, which is soluble at pH 5.5, derived from dimethyl amino ethyl methacrylates and neutral esters of methacrylic acid (Eudragit E). The coating materials are dissolved in a volatile organic solvent (acetone) and the active ingredient is suspended in solution. The solvent is then evaporated and the microcapsules are recovered.

US patent 5,762,961 describes a method for obtaining readily disaggregable porous tablets using an active ingredient, a diluent and a binder in combination with readily volatilisable ammoniacal salts by subsequent heating of the tablets obtained under vacuum in such a way as to obtain a readily disaggregable porous mass.

US patent 5,738,875 describes a process for masking the unpleasant taste of some active ingredients by means of suspension/ dissolution of the active ingredient in an aqueous solution of water-soluble excipients and natural polymers (gelatin) and subsequent lyophilisation of individual dosage units.

US patent 5,837,277 describes a process for masking the unpleasant taste of some active ingredients by subsequent coatings in a fluid bed with aqueous dispersions of formulations based on methacrylic polymers having different permeability properties.

US patent 5,869,098 describes compositions which are useful for producing tablets which can be formed using conventional tabletting machines and which disaggregate rapidly in the mouth. The compositions typically comprise partly hygroscopic matrices which can be recrystalised using crystallisation promoters.

US patent 5,876,759 describes the formation of tablets comprising an active ingredient coated with a mixture of polymers comprising a first polymer selected from cellulose acetate and cellulose acetate butyrate and a second polymer selected from polyvinylpyrrolidone and hydroxypropylcellulose, a disaggregating agent such as mannitol, sorbitol, dextrose, sucrose, xylitol and lactose, and a binder such as cellulose, polyvinylpyrrolidone, starch and modified starch.

US patent 5,866,163 describes the preparation of dissolvable tablets by using an equipment designed for the preparation of a sugar crystalline matrix.

US patent 6,024,981 describes the preparation of tablets which will dissolve in the oral cavity having a friability equal to or lower than 2% and a hardness higher than 15 Newtons.

US patent 6,106,861 claims a process for obtaining rapidly disaggregable tablets (< 40 sec) comprising at least one disaggregating agent such as cross-linked polyvinylpyrrolidone, also known as crospovidone, and cross-linked carboxymethylcellulose, also known as croscarmellose, at least one diluent having binding properties such as mannitol, xylitol, sorbitol and maltitol, and an active ingredient in the form of coated microcrystals. Microcrystal coating of the active ingredient takes place in a fluid bed using polymethacrylates and/or cellulose polymers.

US patent 6,465,009 describes a process for obtaining a rapidly disaggregable tablet comprising a step of granulating the active ingredient and a saccharide excipients with a water-soluble polymer (PVP) which is free from residual solvents, a compression step, and two post-treatments in a humidified atmosphere and subsequent drying.

WO 99/44580 discloses a fast disintegrating tablet comprising a drug in multiparticulate form, in which the drug can be coated with an outer substantially water-insoluble membrane or layer.

### SUMMARY OF THE INVENTION

This invention intends to provide an oral pharmaceutical form which is also suitable for the administration of a pain-relieving product such as, for example, flurbiprofen and ibuprofen. A ready release of the active ingredient is therefore required to these pharmaceutical forms. Orally dispersible tablets or granulates have this feature but also have the disadvantage that the ready release of the active ingredient in the mouth makes it unsuitable for those active ingredients which have a very unpleasant taste such as, for example, flurbiprofen and ibuprofen.

This invention also intends to provide a method for the preparation of an orally dispersible solid pharmaceutical form and an orally dispersible solid pharmaceutical form which readily releases the drug and which has good palatability even when the active ingredient has a very unpleasant taste.

These problems have been overcome by the method and the orally dispersible solid pharmaceutical form according to this invention.

In a first aspect this invention provides a process for the preparation of an orally dispersible solid pharmaceutical form comprising the following steps:
a. coating the active ingredient with at least one hydrophilic carboxylate polymer,
b. granulating the coated active ingredient obtained in step (a) with at least one lipid compound having a melting point lower than that of the active ingredient,
c. mixing the granulate obtained in step (b) with at least one hydrophilic natural polymer having high molecular weight, and
d. mixing the granulate obtained in step (c) with ingredients suitable for obtaining an orally dispersible solid pharmaceutical form.

In a second aspect this invention provides an orally dispersible solid pharmaceutical form comprising an active ingredient coated with at least one hydrophilic carboxylate polymer and at least one lipid compound in which said coated active ingredient is embedded in a matrix comprising at least one hydrophilic natural polymer having high molecular weight.

In this description and the appended claims the term "orally dispersible" is intended to mean any solid administration unit which disaggregates spontaneously in the presence of water or saliva, such a disaggregation being possibly improved by chewing or dispersion, in less than 1.5 minutes, preferably in less than 1 minute, and even more preferably in less than 30 seconds.

In tum, the expression "hydrophilic natural polymer having high molecular weight" is intended to mean a hydrophilic polymer that has been obtained from a plant or an animal and has a molar weight higher than 1,000. Preferebly, higher than 10,000 and, still preferably higher than 100,000.

### DETAILED DESCRIPTION OF THE INVENTION

This invention relates to a process for the preparation of an orally dispersible solid pharmaceutical form comprising the following steps:
a) coating the active ingredient with at least one hydrophilic carboxylate polymer,
b) granulating the coated active ingredient obtained in step (a) with at least one lipid compound having a melting point lower than that of the active ingredient,
c) mixing the granulate obtained in step (b) with at least one hydrophilic natural polymer having high molecular weight, and
d) mixing the granulate obtained in step (c) with ingredients suitable for obtaining an orally dispersible solid pharmaceutical form.

In a preferred aspect of this invention the solid pharmaceutical form is a tablet or a granulate.

Therefore, where it is desired to obtain tablets which will disperse in the mouth, the method according to this invention preferably comprises a further step (e) of compression of the formulation obtained in step (d).

In the case of a granulate, this may be directly subdivided into dosage units and packed in sachets or any other suitable type of packaging.

In this description and the appended claims, granules of active ingredient coated with at least one hydrophilic carboxylate polymer will be identified by the name of "microcapsules".

The microcapsules according to this invention mask the taste of the active ingredient, coated in this way, but allow ready release of the same.

Preferably, while making it possible to mask the taste of the active ingredient, step a) makes possible to obtain a rate of dissolution of the active ingredient which is greater than the dissolution of the active ingredient as such.

Subsequent step b) substantially improves the palatability of the preparation. This step may include a reduction in the rate of dissolution of the active ingredient without however going below the rate of dissolution of the active ingredient as such.

Preferably step c) further improves masking of the taste of the active ingredient without altering its rate of release.

The main purpose of subsequent steps d) and e) is to mix the various components together by direct mixing, and subsequent compression thereof by direct compression where orally dispersible tablets are being prepared, or to subdivide them into dosing units for the subsequent packaging step where orally dispersible granulates are being prepared.

Any active ingredient having an unpleasant taste may be used in connection with this invention. Preferably this invention is used to improve the palatability of non-steroidal anti-inflammatory active ingredients (known also by the acronym NSAID, "Non Steroidal Anti Inflammatory Drug"), such as, for example, salicylic acid derivatives such as, for example, salicylamide, sodium salicylate, aspirin, mesalamine, sulfasalazine, and methyl salicylate, pyrazolone derivatives such as, for example, phenylbutazone, oxyphenbutazone, antipyrine, aminopyrine, dipyrone and apazone (azapropazone), para-aminophenol derivatives such as, for example, phenacetin and acetaminophen (paracetamol), derivatives of N-phenylanthranylic acid, known as fenamates, such as for example mefenamic acid, meclofenamic acid, flufenamic acid, tolfenamic acid, etofenamic acid, and their pharmaceutically acceptable salts, and propionic acid derivatives such as, for example, ibuprofen, naproxen, flurbiprofen, phenoprofen, ketoprofen, phenbufen, pirprofen, oxaprozine, indoprofen and tiaprofenic acid. Each of the abovementioned NSAID is extensively described in the literature as, for example, in Goodman and Gilman, The Pharmacological Basis of Therapeutics (8th edition), McGraw-Hill, 1993, pages 638-381, in the Merck Index, 12th Edition 1996, Merck & Co. Inc., New Jersey, USA, in Martindale, The Complete Drug Reference, the Royal Pharmaceutical Society of Great Britain, 1 Lambeth High Street, London SE1 7JN, UK, and in the US, British and European Pharmacopoeias.

Typically, the active ingredient is ibuprofen, naproxen or flurbiprofen in the water-insoluble acid form.

The hydrophilic carboxylate polymer which can be used according to this invention is selected from the group comprising carboxyalkylcellulose polymers such as, for example, carboxymethylcellulose and carboxypropylcellulose, hemiesters of dicarboxylic acids with alkyl cellulose such as, for example, hydroxypropylmethylcellulose phthalate, hydroxypropylmethylcellulose succinate, and cellulose acetophthalate, and copolymers of an alkenyl carboxy acid with alkyl esters of an alkenyl carboxy acid such as, for example, acrylic and methacrylic acid and/or acrylate and/or methacrylate copolymers. The polymer which is preferred for use in this invention is hyroxypropylmethylcellulose phthalate, in particular the commercial products HP-50™ and HP-55™ from the company Shin-Etsu Chemical Co., Ltd, Japan. These commercial products are characterised in that they contain hydroxypropyl residues (from 5 to 10 molar %), methoxy residues (from 18 to 24 molar %), phthalyl residues (from 21 to 35 molar %) and by their molecular weight (about 80,000 ± 10,000).

The preferred hydrophilic carboxylate polymer according to this invention is selected from the group comprising hemiesters of dicarboxylic acids with alkyl cellulose.

Typically, said hydrophilic carboxylate polymer is hydroxypropylmethylcellulose phthalate or succinate.

Preferably, the weight ratio between the active ingredient and said hydrophilic carboxylate polymer is comprised between 60:40 and 99.9 : 0.1. More preferably, said ratio ranges from 75:25 to 99:1. Typically, this ranges from 85:15 to 95:5. Therefore, the parts by weight of hydrophilic carboxylate polymer added for each part by weight of active ingredient preferably range from 0.67 to 0.001, more preferably from 0.33 to 0.01, even more preferably from 0.175 to 0.05.

The lipid compound which may be used according to this invention is selected from the group comprising fatty acids such as, for example, stearic acid, esters of fatty acids with aliphatic alcohols such as, for example, glyceryl dibehenate, glyceryl distearate and glyceryl palmitostearate (Precirol^{™} ATO5 produced and distributed by Gattofossé Milano, Italy), fatty alcohols such as, for example, cetyl alcohol, cetostearyl alcohol, stearyl alcohol, oleyl alcohol and myristyl alcohol, and triglycerides of fatty acids such as, for example, the commercial products Supocire^{™} produced and distributed by Gattofossé Italia, Milan, and Witepsol^{™} produced and distributed by Condea Chemie GmbH, Germany, and mixtures thereof.

Preferably, the lipid compound according to this invention is selected from the group comprising aliphatic alcohols having 12 to 18 carbon atoms. Typically, the lipid compound according to this invention is cetyl alcohol.

Preferably, the weight ratio between the active ingredient and said lipid compound is comprised between 75:25 and 99.9 : 0.1. More preferably, said ratio ranges from 80:20 to 99:1. Typically, this ranges from 85:15 to 95:5. Thus, the parts by weight of lipid compound added for each part by weight of active ingredient preferably range from 0.33 to 0.001, more preferably from 0.25 to 0.01, even more preferably again from 0.175 to 0.05.

The hydrophilic natural polymer having high molecular weight which may be used according to this invention is preferably selected from the group comprising guar gum, arabic gum, karaya gum, gelano gum, carrageenan, chitosan, galactan, Polglumyt^{™} (the trade name of a deproteinated fraction of glycogen produced and distributed by A.C.R.A.F. S.p.A. Rome, Italy, and described in patent EP 654,048), and mixtures thereof.

The particularly preferred hydrophilic natural polymer having high molecular weight according to this invention is guar gum.

Preferably the weight ratio between the active ingredient and the hydrophilic natural polymer having high molecular weight (or mixtures thereof) is comprised between 75:25 and 99.9 : 0.1. More preferably, said ratio ranges from 80:20 to 99.5 : 0.5. Typically this ranges from 85:15 to 99:1. Thus the parts by weight of hydrophilic natural polymer having high molecular weight (or mixtures thereof) added for each part by weight of the active ingredient preferably range from 0.33 to 0.001, more preferably from 0.25 to 0.005, even more preferably again from 0.175 to 0.01.

In a preferred embodiment of this invention, in particular in the case where orally dispersible tablets are prepared, the hydrophilic natural polymer having high molecular weight is used as a mixture with microcrystalline cellulose to improve the compressibility of the mixture. A mixture containing 1 to 15 parts by weight of microcrystalline cellulose for each part by weight of guar gum is particularly preferred. A mixture containing 4 to 10 parts by weight of microcrystalline cellulose for each part by weight of guar gum is even more preferred.

When the hydrophilic natural polymer having high molecular weight is added to a mixture with microcrystalline cellulose, the parts by weight of that mixture added for each part by weight of the active ingredient range from 1.2 to 0.1. More preferably said range ranges from 1.0 to 0.2.

Other ingredients which may be used according to this invention comprise diluents, exfoliating agents, disaggregating agents, sweeteners, flavourings, lubricants and the like.

Examples of suitable diluents comprise lactose, starch, mannitol, dextrose, calcium silicate, sorbitol, xylitol.

Examples of suitable exfoliating agents comprise mannitol, dextrose, calcium silicate.

Examples of suitable disaggregating agents comprise AcDiSol (sodium croscarmellose), polyplasdone (cross-linked PVP), Explotab^{™} (sodium starch glycolate).

Examples of suitable sweeteners comprise aspartame, saccharine, acesulfame.

Examples of suitable flavourings comprise grapefruit flavour, raspberry flavour, lemon flavour, orange flavour and the like.

Examples of suitable lubricants comprise colloidal silica, magnesium stearate, PEG4000, PEG6000, PEG20000, sodium benzoate, sodium acetate, sodium oleate, magnesium lauryl sulphate.

In the method according to this invention the step of coating the active ingredient with at least one hydrophilic carboxylate polymer is preferably carried out by means of the microencapsulation technique. The microencapsulation technique comprises coating a finely powdered active ingredient with a compound capable of forming a thin film around the microparticle of active ingredient. The dimensions of the microcapsules so obtained are preferably between 0.5 and 1000 micrometres.

Various methods of microencapsulation are known in the art, such as the interfacial polymerisation method, the *in situ* polymerisation method, the extrusion method, the coacervation method, the solvent evaporation method, the spray method.

A method which can be used to implement the method of this invention is the method described in US patent 4,766,012, in which the hydrophilic carboxylate polymer is dissolved in water by means of a salification process, the particles of the active ingredient are dispersed in water and then added to the hydrophilic carboxylate polymer solution under continuous stirring, then adding an acid compound which causes the hydrophilic carboxylate polymer to precipitate out onto the particles of active ingredient.

In the method according to this invention the step of granulation of the coated active ingredient obtained in the step described above with at least one lipid compound having a melting point lower than that of the active ingredient is carried out according to known techniques, preferably by the rotary granulation technique by means of heatable jacket rotary granulators. Further details will be described in the experimental part.

In the method according to this invention the step of mixing the granulate obtained in the preceding step with at least one hydrophilic natural polymer having high molecular weight in a mixture, optionally, with microcrystalline cellulose, is carried out according to known techniques, preferably by the direct mixing technique with conventional V-shaped mixers or DIOSNA mixers/granulators.

In the method of this invention, the step of mixing the granulate obtained in the preceding step with ingredients suitable for producing orally dispersible tablets or granulates is carried out according to known techniques, preferably by means of the direct mixing technique with conventional V-shaped mixers or DIOSNA mixers/ granulators.

In the method according to this invention the step of compression or subdivision into dose units and subsequent packaging of the formulation obtained in the preceding step is carried out by means of conventional techniques and equipments.

The following examples will illustrate this invention without however in any way restricting it.

### EXPERIMENTAL PART

### Materials and methods

### Dissolution Test

The test was carried out in phosphate buffer at pH 7.2 (obtained by dissolving 6.8 g of KH₂ PO₄ and 1.4 g of NaOH in 1 litre of demineralised water) at 100 rpm for the microcapsule dissolution test and 50 rpm for the tablet dissolution test according to the method described in European Pharmacopoeia, Ed. 4.4 of the 04/2003.

### Evaluation of Palatability

A curtain amount of the product under test was administered to 5 different individuals sensitive to the initating action of ibuprofen. Previously, they had been given a form and they had been asked to indicate on the form their assessment of the palatability of the product administered at the time when it was placed in the mouth (to), during swallowing (t₁), after swallowing (t₂) and after 5 minutes (t₃) on the basis of the following parameters:

| Stimulus: | Description: |
|---|---|
| Burning | Sensation caused by abrasion of the skin, or by exposure to high temperature, or to the irritant action of alcohol; |
| Prickling | Production of a short sensation like an insect bite or pins, |
| Tingling | A sensation similar to that due to the action of small penetrating needles, |
| Dullness | A diffuse sensation like the onset of the action of an anaesthetic (not lack of feeling), |
| Sandiness | Prolonged sensation similar to the presence of grains of sand in the mouth. |

The 5 individuals were also asked to say whether the stimulus was strong (3), medium (2), weak (1) or zero (0).

The palatability of the product under test was therefore worse the higher the abovementioned numerical value associated with the stimulus felt by the individuals subjected to the test.

### Ibuprofen BP 80

Ibuprofen having the following properties:
- free apparent density: 0.328,
- compacted apparent density: 0.505,
- flowability of the active ingredient: 34 mm hole (test carried out using Flowdex^{™} equipment from the company Giuliani Tecnologie Srl, Turin, Italy) using the procedure reccomended for the equipment by the manufacturer,
- particle size: as follows:

| Sieve | Percent |
|---|---|
| 1180 micron (16 mesh) | 0 |
| 850 micron (20 mesh) | 0 |
| 600 micron (30 mesh) | 93.6 |
| 425 micron (40 mesh) | 5.1 |
| 300 micron (50 mesh) | 0.7 |
| 250 micron (60 mesh) | 0.1 |
| 180 micron (80 mesh) | 0.2 |
| 150 micron (100 mesh) | 0.1 |
| 106 micron (140 mesh) | 0.1 |
| 75 micron (200 mesh) | 0 |
| 53 micron (270 mesh) | 0 |
| 38 micron (400 mesh) | 0 |
| Residue | 0.1 |

- dissolution in phosphate buffer at pH 7.2 (obtained by dissolving 6.8 g of KH₂PO₄ and 1.4 g of NaOH in 1 litre of demineralised water) at 100 rpm; the average of 5 tests has given the following results: 30.1% after 5 minutes, 58.6% after 10 minutes, 71.0% after 15 minutes, 78.8% after 20 minutes, 86.9% after 30 minutes,
- palatability: the palatability test was carried out as indicated above administering 200 mg of Ibuprofen BP 80 to each individual. The results are shown in the table below and show that the palatability of ibuprofen as such is very bad.

| Individual | Burning | | | | Prickling | | | | Tingling | | | | Dullness | | | | Sandiness | | | | Total |
|---|---|---|---|---|---|---|---|---|---|---|---|---|---|---|---|---|---|---|---|---|---|
| | t₀ | t₁ | t₂ | t₃ | t₀ | t₁ | t₂ | t₃ | t₀ | t₁ | t₂ | t₃ | t₀ | t₁ | t₂ | t₃ | t₀ | t₁ | t₂ | t₃ | |
| 1 | 3 | 3 | 2 | 1 | 3 | 3 | 2 | 0 | 3 | 2 | 2 | 2 | 3 | 2 | 1 | 0 | 2 | 1 | 0 | 0 | 35 |
| 2 | 3 | 3 | 3 | 3 | 3 | 3 | 3 | 2 | 3 | 3 | 3 | 1 | 3 | 3 | 2 | 1 | 1 | 1 | 0 | 0 | 44 |
| 3 | 3 | 3 | 2 | 1 | 3 | 2 | 1 | 1 | 3 | 2 | 1 | 0 | 2 | 2 | 1 | 0 | 2 | 1 | 0 | 0 | 30 |
| 4 | 3 | 3 | 3 | 2 | 3 | 3 | 3 | 2 | 3 | 3 | 2 | 1 | 3 | 3 | 2 | 1 | 1 | 1 | 0 | 0 | 42 |
| 5 | 3 | 3 | 1 | 1 | 3 | 3 | 1 | 0 | 3 | 3 | 1 | 0 | 2 | 2 | 1 | 0 | 1 | 1 | 0 | 0 | 29 |

### Hydrophilic Carboxylate Polymer

A hydroxypropylmethylcellulose phthalate having the trade name HP-55™ from the Japanese company Shin-Etsu Chemical Co. Ltd. was used.

### Abbreviations

In the examples below, the following abbreviations have the following meanings:
Al = Active Ingredient
HCP = Hydrophilic Carboxylate Polymer
LC = Lipid Compound
MC1 = Microcapsules of Example 1
MC2 = Microcapsules of Example 2
MC3 = Microcapsules of Example 3
GR1 = Granulate 1
GR2 = Granulate 2
GR3 = Granulate 3
GR4 = Granulate 4

### Ratios

Where not indicated otherwise, the ratios between AI and HCP or LC are intended to be ratios by weight.

### Example 1

Preparation of Microcapsules (AI: HCP = 95:5) 95.00 g of Ibuprofen BP 80, 5.00 g of HP-55™ polymer and 0.75 g of KOH were used.

A solution of potassium hydroxide in demineralised water (about 60 ml) was prepared in a first container. The HP-55™ was added to that solution until completely dissolved. In another container a suspension of Ibuprofen BP 80 was prepared in demineralised water (500 ml) and the suspension so obtained was homogenised. The solution was then added to the suspension under continuous stirring. The so obtained mixture was made acid with 1 N HCl, maintaining stirring at all times, until a pH value of from 2.5 to 2.9 was obtained. The microcapsules which precipitated out during acidification were collected on a filter and then manually placed on a tray.

The so obtained particles were dried in a static bed oven by forced hot air at 40°C for some hours. Finally, the particles were placed on a 30 mesh (600 micron) sieve and those which passed through that sieve were collected.

### Example 2

### Preparation of Microcapsules (AI: HCP = 90:10)

The preparation was carried out in a similar way to that described in Example 1 above by using 630.00 g of Ibuprofen BP 80, 70.00 g of HP-55™, 11.00 g of KOH, 0.63 g of saccharose monopalmitate and 1.26 g of dimethicone.

A solution of potassium hydroxide in demineralised water (about 820 ml) was prepared in a first container and then HP-55™ was added until complete dissolution. A suspension of the active ingredient in demineralised water (about 3500 ml) to which the saccharose monopalmitate and the dimethicone had previously been added was prepared in another container and the suspension so obtained was homogenised.

The procedure was then continued as described in Example 1.

### Example 3

### Preparation of Microcapsules (AI: HCP = 75:25)

The preparation was carried out in a similar way to that described in Example 2 above using 525.00 g of ibuprofen BP 80,175.00 g of HP-55™, 26.80 g of KOH, 0.54 g of saccharose monopalmitate and 1.08 g of dimethicone.

### Example 4

### Microcapsule Releasing Test

The microcapsules obtained in Examples 1 to 3 (MC1, MC2 and MC3) were subjected to the releasing test in phosphate buffer by means of a UV spectrophotometer. The percentages of AI released between 0 and 45 minutes are shown in the table below. In order to assist comparison the results obtained on AI as such in the same test are shown in the bottom line (see previous Experimental Part).

| | 0 min | 5 min | 10 min | 15 min | 20 min | 25 min | 30 min | 35 min | 40 min | 45 min |
|---|---|---|---|---|---|---|---|---|---|---|
| MIC1 | 0 | 63.1 | 100.00 | 100.00 | 100.00 | 100.00 | 100.00 | 100.00 | 100.00 | 100.00 |
| MIC2 | 0 | 64.83 | 94.13 | 97.63 | 98.00 | - | 98.00 | - | - | 98.10 |
| MIC3 | 0 | 64.00 | 94.33 | 97.43 | 98.23 | - | 98.47 | - | - | 98.67 |
| AI | 0 | 30.10 | 58.60 | 71.00 | 78.80 | - | 86.90 | | | |

The data in the table above show that coating the AI with HCP clearly improves releasing times for the active ingredient as if the HCP was acting as a surfactant. The effect of the HCP was substantially the same for all experimental quantities of HCP.

### Example 5

### Palatability of Microcapsules

Microcapsules MC1, MC2 and MC3 were subjected to the palatability test carried out with the same persons and the same procedures as described previously, with the administration of an amount of microcapsules containing 200 mg of the active ingredient. The results are shown in the tables below.

**MC1**

| Individual | Burning | | | | Prickling | | | | Tingling | | | | Dullness | | | | Sandiness | | | | Total |
|---|---|---|---|---|---|---|---|---|---|---|---|---|---|---|---|---|---|---|---|---|---|
| | t₀ | t₁ | t₂ | t₃ | t₀ | t₁ | t₂ | t₃ | t₀ | t₁ | t₂ | t₃ | t₀ | t₁ | t₂ | t₃ | t₀ | t₁ | t₂ | t₃ | |
| 1 | 3 | 2 | 2 | 1 | 3 | 2 | 2 | 0 | 2 | 2 | 2 | 2 | 2 | 2 | 1 | 0 | 1 | 3 | 3 | 1 | 36 |
| 2 | 3 | 3 | 2 | 1 | 3 | 3 | 2 | 2 | 2 | 3 | 2 | 1 | 2 | 2 | 2 | 1 | 1 | 2 | 2 | 2 | 41 |
| 3 | 2 | 3 | 1 | 1 | 2 | 2 | 1 | 1 | 3 | 2 | 1 | 0 | 2 | 2 | 0 | 0 | 1 | 3 | 3 | 1 | 31 |
| 4 | 3 | 3 | 2 | 0 | 3 | 3 | 2 | 0 | 2 | 3 | 2 | 1 | 3 | 2 | 1 | 1 | 1 | 3 | 2 | 1 | 38 |
| 5 | 2 | 3 | 1 | 1 | 2 | 3 | 1 | 0 | 2 | 3 | 1 | 0 | 2 | 2 | 1 | 0 | 1 | 2 | 2 | 1 | 30 |

**MC2**

| Individual | Burning | | | | Prickling | | | | Tingling | | | | Dullness | | | | Sandiness | | | | Total |
|---|---|---|---|---|---|---|---|---|---|---|---|---|---|---|---|---|---|---|---|---|---|
| | t₀ | t₁ | t₂ | t₃ | t₀ | t₁ | t₂ | t₃ | t₀ | t₁ | t₂ | t₃ | t₀ | t₁ | t₂ | t₃ | t₀ | t₁ | t₂ | t₃ | |
| 1 | 1 | 2 | 2 | 1 | 2 | 2 | 2 | 0 | 2 | 2 | 2 | 2 | 1 | 2 | 1 | 0 | 1 | 3 | 2 | 0 | 30 |
| 2 | 2 | 2 | 2 | 1 | 2 | 2 | 2 | 1 | 2 | 2 | 2 | 1 | 1 | 2 | 2 | 0 | 0 | 2 | 2 | 0 | 30 |
| 3 | 2 | 2 | 1 | 1 | 1 | 2 | 1 | 1 | 2 | 2 | 1 | 1 | 1 | 2 | 0 | 0 | 1 | 2 | 2 | 0 | 25 |
| 4 | 1 | 2 | 2 | 0 | 1 | 3 | 2 | 0 | 1 | 2 | 2 | 1 | 2 | 2 | 1 | 1 | 1 | 3 | 2 | 0 | 29 |
| 5 | 2 | 2 | 1 | 1 | 1 | 3 | 1 | 0 | 2 | 3 | 1 | 0 | 1 | 2 | 1 | 0 | 0 | 3 | 2 | 0 | 26 |

**MC3**

| Individual | Burning | | | | Prickling | | | | Tingling | | | | Dullness | | | | Sandiness | | | | Total |
|---|---|---|---|---|---|---|---|---|---|---|---|---|---|---|---|---|---|---|---|---|---|
| | t₀ | t₁ | t₂ | t₃ | t₀ | t₁ | t₂ | t₃ | t₀ | t₁ | t₂ | t₃ | t₀ | t₁ | t₂ | t₃ 0 | t₀ | t₁ | t₂ | t₃ | |
| 1 | 1 | 2 | 2 | 1 | 2 | 2 | 2 | 0 | 2 | 2 | 2 | 2 | 1 | 2 | 1 | 0 | 1 | 3 | 2 | 0 | 30 |
| 2 | 2 | 2 | 2 | 1 | 2 | 2 | 2 | 1 | 2 | 2 | 2 | 1 | 1 | 2 | 2 | 0 | 0 | 2 | 2 | 0 | 30 |
| 3 | 2 | 2 | 1 | 1 | 1 | 2 | 1 | 1 | 2 | 2 | 1 | 1 | 1 | 2 | 0 | 0 | 1 | 2 | 2 | 0 | 25 |
| 4 | 1 | 2 | 2 | 0 | 1 | 3 | 2 | 0 | 1 | 2 | 2 | 1 | 2 | 2 | 1 | 1 | 1 | 3 | 2 | 0 | 29 |
| 5 | 2 | 2 | 1 | 1 | 1 | 3 | 1 | 0 | 2 | 3 | 1 | 0 | 1 | 2 | 1 | 0 | 0 | 3 | 2 | 0 | 26 |

The data shown in the tables above indicate that coating the AI with HCP partly masked the unpleasant taste of the AI, especially in the case of MC2 and MC3.

### Example 6

### Preparation Granulates

MC1 and MC2 were granulated with cetyl alcohol as the lipid compound (LC) in order to increase the taste-masking effect and improve compressibility. Granulation was carried out in a Rotolab rotary granulator from the Zanchetta company, Lucca, Italy.

The following granulates were prepared in this way:

| | GR1 | GR2 | GR3 | GR4 |
|---|---|---|---|---|
| MC1 (g) | 500.00 | 550.00 | - | - |
| MC2 (g) | - | - | 550.00 | 500.00 |
| Cetyl alcohol (g) | 55.50 | 28.95 | 28.95 | 55.50 |
| Total (g) | 550.50 | 578.95 | 578.95 | 555.50 |

The preparation of each granulate was carried out by setting the jacket temperature of the rotary granulator to a value such that softening of the LC was attained without however damaging the structure of the microcapsules. In the particular cases of Granulates 1-4 the jacket temperature was set to 50°C.

Then, the microcapsules and the cetyl alcohol which had previously been milled and passed through a 30 mesh (600 µm) sieve were poured into the chamber of the rotary granulator. The powders were mixed for at least 180 seconds, with the mixing paddle speed set to 700 rpm. Heating of the granulator jacket was then begun, setting the temperature to 50°C. Stirring was continued until the product temperature reached 47°C.

The granulation process was interrupted a few minutes after that said temperature had been reached.

Finally, after cooling to room temperature the granulate so obtained was sifted on a 20 mesh (850 µm) sieve.

Palatability of the granulate is further improved when the granulation process is carried out as follows:
1) heating under stirring the whole amount of cetyl alcohol up to melting and subsequent cooling;
2) suspending MC1, MC2 or MC3 in a reactor provided with a stirring blade;
3) adding cetyl alcohol from step 1) to the suspension of step 2) and subsequent mixing;
4) cooling the suspension obtained in step 3);
5) filtering the suspension obtained in fase 4).

### Example 7

### Releasing Test on Granulates

A releasing test in phosphate buffer of from 0 to 30 minutes was carried out on four granulates GR1-GR4 by means of HPLC analysis.

The results shown in the table below are percentages by weight.

| | 0 min | 5 min | 10 min | 15 min | 20 min | 30 min |
|---|---|---|---|---|---|---|
| GR1 | 0 | 21.0 | 38.7 | 51.7 | 62.0 | 72.0 |
| GR2 | 0 | 46.5 | 71.5 | 83.4 | 89.2 | 94.1 |
| GR3 | 0 | 46.6 | 64.5 | 75.6 | 82.9 | 91.0 |
| GR4 | 0 | 45.7 | 73.2 | 80.3 | 85.0 | 90.0 |

The above results show that cetyl alcohol has some retarding action on the rate of release of the active ingredient. Good results were however obtained with granulates GR2 and GR4.

### Example 8

### Palatability of Granulates

Granulates GR1-GR4 were subjected to the palatability test carried out with the same persons and the same procedures as described previously with the administration of an amount of granulate containing 200 mg of active ingredient. The results are shown in the tables below.

**GR1**

| Individual | Burning | | | | Prickling | | | | Tingling | | | | Dullness | | | | Sandiness | | | | Total |
|---|---|---|---|---|---|---|---|---|---|---|---|---|---|---|---|---|---|---|---|---|---|
| | t₀ | t₁ | t₂ | t₃ | t₀ | t₁ | t₂ | t₃ | t₀ | t₁ | t₂ | t₃ | t₀ | t₁ | t₂ | t₃ | t₀ | t₁ | t₂ | t₃ | |
| 1 | 0 | 1 | 1 | 1 | 0 | 1 | 1 | 0 | 1 | 1 | 1 | 1 | 0 | 1 | 1 | 0 | 0 | 2 | 2 | 0 | 15 |
| 2 | 0 | 1 | 1 | 1 | 0 | 1 | 1 | 1 | 0 | 1 | 1 | 1 | 0 | 1 | 1 | 0 | 1 | 3 | 3 | 0 | 18 |
| 3 | 0 | 1 | 1 | 1 | 0 | 1 | 1 | 1 | 1 | 2 | 1 | 1 | 0 | 1 | 0 | 0 | 1 | 2 | 2 | 0 | 17 |
| 4 | 0 | 1 | 0 | 0 | 0 | 1 | 1 | 0 | 0 | 2 | 2 | 1 | 1 | 1 | 1 | 0 | 0 | 3 | 2 | 0 | 16 |
| 5 | 0 | 1 | 1 | 1 | 0 | 2 | 2 | 0 | 0 | 1 | 1 | 0 | 0 | 1 | 1 | 0 | 1 | 3 | 2 | 0 | 17 |

**GR2**

| Individual | Burning | | | | Pickling | | | | Tingling | | | | Dullness | | | | Sandiness | | | | Total |
|---|---|---|---|---|---|---|---|---|---|---|---|---|---|---|---|---|---|---|---|---|---|
| | t₀ | t₁ | t₂ | t₃ | t₀ | t₁ | t₂ | t₃ | t₀ | t₁ | t₂ | t₃ | t₀ | t₁ | t₂ | t₃ | t₀ | t₁ | t₂ | t₃ | |
| 1 | 1 | 2 | 2 | 1 | 0 | 1 | 1 | 0 | 1 | 2 | 1 | 1 | 0 | 1 | 1 | 1 | 1 | 2 | 3 | 0 | 22 |
| 2 | 1 | 2 | 1 | 1 | 0 | 1 | 1 | 1 | 1 | 1 | 1 | 1 | 0 | 1 | 1 | 1 | 1 | 3 | 3 | 0 | 22 |
| 3 | 1 | 2 | 1 | 1 | 0 | 1 | 1 | 1 | 1 | 2 | 1 | 1 | 0 | 1 | 0 | 1 | 1 | 2 | 2 | 0 | 20 |
| 4 | 0 | 2 | 1 | 0 | 0 | 1 | 1 | 0 | 1 | 2 | 2 | 1 | 1 | 1 | 1 | 0 | 2 | 2 | 2 | 0 | 20 |
| 5 | 0 | 2 | 1 | 1 | 0 | 2 | 2 | 0 | 1 | 1 | 2 | 1 | 0 | 1 | 1 | 1 | 0 | 3 | 2 | 1 | 22 |

**GR3**

| Individual | Burning | | | | Prickling | | | | Tingling | | | | Dullness | | | | Sandiness | | | | Total |
|---|---|---|---|---|---|---|---|---|---|---|---|---|---|---|---|---|---|---|---|---|---|
| | t₀ | t₁ | t₂ | t₃ | t₀ | t₁ | t₂ | t₃ | t₀ | t₁ | t₂ | t₃ | t₀ | t₁ | t₂ | t₃ | t₀ | t₁ | t₂ | t₃ | |
| 1 | 1 | 2 | 1 | 1 | 2 | 2 | 1 | 0 | 2 | 2 | 2 | 1 | 1 | 2 | 1 | 0 | 0 | 2 | 2 | 0 | 25 |
| 2 | 2 | 2 | 1 | 1 | 2 | 2 | 1 | 1 | 2 | 2 | 2 | 1 | 1 | 2 | 1 | 0 | 1 | 3 | 2 | 1 | 30 |
| 3 | 2 | 2 | 1 | 1 | 1 | 2 | 1 | 1 | 2 | 2 | 1 | 0 | 1 | 2 | 0 | 0 | 0 | 2 | 2 | 0 | 23 |
| 4 | 1 | 2 | 1 | 0 | 1 | 2 | 2 | 0 | 1 | 2 | 1 | 1 | 2 | 2 | 1 | 1 | 1 | 2 | 2 | 0 | 25 |
| 5 | 2 | 2 | 1 | 1 | 1 | 3 | 1 | 0 | 2 | 3 | 1 | 0 | 1 | 2 | 1 | 0 | 1 | 2 | 2 | 1 | 27 |

**GR4**

| Individual | Burning | | | | Prickling | | | | Tingling | | | | Dullness | | | | Sandiness | | | | Total |
|---|---|---|---|---|---|---|---|---|---|---|---|---|---|---|---|---|---|---|---|---|---|
| | t₀ | t₁ | t₂ | t₃ | t₀ | t₁ | t₂ | t₃ | t₀ | t₁ | t₂ | t₃ | t₀ | t₁ | t₂ | t₃ | t₀ | t₁ | t₂ | t₃ | |
| 1 | 1 | 2 | 1 | 0 | 1 | 2 | 0 | 0 | 1 | 2 | 1 | 1 | 1 | 2 | 1 | 0 | 0 | 2 | 2 | 0 | 20 |
| 2 | 1 | 1 | 1 | 1 | 1 | 2 | 1 | 1 | 2 | 2 | 2 | 0 | 1 | 1 | 1 | 0 | 1 | 3 | 3 | 0 | 25 |
| 3 | 1 | 2 | 1 | 0 | 1 | 2 | 0 | 0 | 1 | 2 | 1 | 0 | 1 | 1 | 0 | 0 | 1 | 2 | 3 | 1 | 20 |
| 4 | 1 | 1 | 1 | 0 | 0 | 2 | 1 | 0 | 1 | 2 | 1 | 1 | 1 | 2 | 1 | 0 | 1 | 2 | 3 | 0 | 21 |
| 5 | 1 | 2 | 1 | 1 | 1 | 3 | 1 | 0 | 1 | 3 | 1 | 0 | 1 | 2 | 1 | 0 | 1 | 2 | 2 | 0 | 24 |

The above data show excellent palatability values for GR1 and acceptable values for GR2 and GR4.

### Example 9

### Preparation of Orally Dispersible Tablets

A first batch of tablets having the following weight percentage composition was prepared:

| Ingredient | | % |
|---|---|---|
| A. GR4 | : | 17.40 |
| B. Microcrystalline cellulose | : | 9.01 |
| C. Guar gum | : | 1.59 |
| D. Colloidal silica | : | 0.84 |
| E. Mannitol | : | 56.30 |
| F. Aspartame | : | 6.00 |
| G. Citric acid | : | 0.70 |
| H. Magnesium stearate | : | 0.84 |
| I. Grapefruit flavour | : | 3.50 |
| J. Ac-Di-Sol™ | : | 3.50 |
| K. Saccharose monopalmitate | : | 0.35 |

Ac-Di-Sol^{™} is the registered trade mark of a product based on sodium croscarmellose manufactured and distributed by the FMC Corporation, Philadelphia, PA, USA.

The preparation was carried out by first mixing A with B and C.

The other ingredients were mixed separately and sifted through a 14 mesh (1400 µm) sieve.

Then, the first mixture of A, B and C was mixed with the second mixture of the other ingredients to obtain a homogeneous dispersion with good flow properties.

Finally, the so obtained mixture was compressed by means of an alternating tabletting machine.

Tablets having an average hardness of 2.4 Kp and a weight of 1.4 g each were produced in this way. Each tablet contained 200 mg of ibuprofen.

### Example 10

### Preparation of Orally Dispersible Tablets

A second batch of tablets having the following weight percentage composition was prepared in the same way as described in Example 9 above:

| Ingredient | | % |
|---|---|---|
| A. GR4 | | 16.80 |
| B. Microcrystalline cellulose | | 8.67 |
| C. Guar gum | | 1.53 |
| D. Colloidal silica | | 0.81 |
| E. Mannitol | | 54.40 |
| F. Aspartame | | 5.78 |
| G. Citric acid | | 0.68 |
| H. Magnesium stearate | | 0.82 |
| I. Orange flavour | | 5.44 |
| J. Raspberry flavour | | 1.36 |
| K. Ac-Di-Sol™ | | 3.40 |
| L. Saccharose monopalmitate | | 0.34 |

### Example 11

### Releasing Test on Orally Dispersible Tablets

The test was carried out at pH 7.2 in a phosphate buffer by means of HPLC analysis on six tablets obtained according to Example 9. The results are shown in the table below and are expressed as percentages by weight of active ingredient released from each tablet from minute 0 to minute 45.

| Tablet | 0 min | 5 min | 10 min | 15 min | 20 min | 30 min | 45 min |
|---|---|---|---|---|---|---|---|
| 1 | 0 | 62.6 | 77.1 | 82.4 | 85.4 | 89.1 | 92.2 |
| 2 | 0 | 58.9 | 75.3 | 81.5 | 85.2 | 89.5 | 93.3 |
| 3 | 0 | 62.3 | 77.0 | 83.1 | 86.6 | 91.1 | 94.8 |
| 4 | 0 | 61.7 | 75.9 | 81.9 | 85.4 | 89.4 | 93.2 |
| 5 | 0 | 62.8 | 76.3 | 82.1 | 85.6 | 89.7 | 93.4 |
| 6 | 0 | 58.8 | 75.5 | 81.8 | 85.9 | 90.2 | 94.5 |

### Example 12

### Palatability of Orally Dispersible Tablets

The palatability test was carried out as described above on a group of 21 persons of different ages and sex, smokers and non-smokers, users and non-users of analgesic drugs, but all sensitive to the irritating action of ibuprofen.

The results shown in the table below are the total average scores for each sensation observed by the individuals (burning, prickling, tingling, dullness, sandiness) resulting from the sum of the scores obtained at times to, t₁, t₂ and t₃ after each administration. The last line shows the total average for all sensations.

| | Example 9 | Example 10 |
|---|---|---|
| Burning | 4.0 ± 0.86 | 3.7 ± 0.82 |
| Pickling | 2.0 ± 0.69 | 1.9 ± 0.70 |
| Tingling | 4.6 ± 0.70 | 1.4 ± 0.59 |
| Dullness | 2.9 ± 0.73* | 3.0 ± 0.78* |
| Sandiness | 3.5 ± 0.68 | 2.7 ± 0.68 |
| Total | 17.0 ± 3.66 | 15.7 ± 3.57 |

The results of Examples 11 and 12 showed the good palatability results obtained with the tablets of Example 9 and, even better, with those of Example 10, while maintaining the property of readily releasing the active ingredient.

### Example 13

### Preparation of Orally Dispersible Granulates

A granulate having the following weight percentage composition was prepared:

| Ingredient | % | |
|---|---|---|
| A. GR1 | | 19.00 |
| B. Microcrystalline cellulose | | 2.60 |
| C. Guar gum | | 0.50 |
| D. Aspartame | | 3.10 |
| E. Citric acid | | 2.50 |
| F. Cola flavour | | 4.60 |
| G. Dextrose | | 63.10 |
| H. Sodium bicarbonate | | 4.60 |

The preparation was carried out by first mixing A with B and C.

The other ingredients were mixed separately and sifted through a 14 mesh (1400 µm) sieve.

The first mixture of A, B and C was then mixed with the second mixture of the other ingredients for a total time of 10 minutes to obtain a homogeneous dispersion with good flow properties.

By working in the same way, two other granulates containing, , grapefruit flavour and orange/raspberry flavour instead of the cola flavour were prepared. The palatability test on the granulates so obtained, carried out with the same persons and the same procedures as described previously and by administering an amount of granulate containing 200 mg of active ingredient, showed results which were better than or comparable with the palatability results obtained with the tablets.

### Example 14

### Releasing Test on Orally Dispersible Granulates

The test was carried out in phosphate buffer at pH 7.2 by means of HPLC analysis on granulates obtained according to Example 13. The results are shown in the table below and are expressed as percentages by weight of active ingredient released from each tablet between minute 0 and minute 15.

| Granulate | 0 min | 2 min | 4 min | 6 min | 8 min | 15 min |
|---|---|---|---|---|---|---|
| 1 | 0 | 62.5 | 75.1 | 86.3 | 93.2 | 95.7 |
| 2 | 0 | 61.6 | 79.4 | 84.4 | 94.6 | 99.7 |
| 3 | 0 | 67.2 | 77.7 | 84.8 | 85.2 | 98.8 |
| 4 | 0 | 62.3 | 78.6 | 84.5 | 90.1 | 100.0 |
| 5 | 0 | 65.2 | 68.3 | 80.5 | 83.5 | 94.9 |
| 6 | 0 | 67.8 | 83.2 | 87.2 | 92.1 | 100.0 |

The results in the table confirmed that the granulate has a good ability of readily releasing the active ingredient.

## Claims

1. A process for the preparation of an orally dispersible solid pharmaceutical form **characterised in that** it comprises the following steps:
a. coating the active ingredient with at least one hydrophilic carboxylate polymer,
b. granulating the coated active ingredient obtained in step (a) with at least one lipid compound having a melting point lower than that of the active ingredient,
c. mixing the granulate obtained in step (b) with at least one hydrophilic natural polymer having high molecular weight, and
d. mixing the granulate obtained in step (c) with ingredients suitable for obtaining an orally dispersible solid pharmaceutical form.

2. A process according to Claim 1, **characterised in that** said solid pharmaceutical form is selected from tablets and granulates.

3. A process according to Claim 1, **characterised in that** after step (d) it comprises the step of:
e) compressing the formulation obtained in step d) to obtain orally dispersible tablets.

4. A process according to Claim 1, **characterised in that** after step (d) it comprises the step of:
e) subdividing the formulation obtained in step (d) to obtain dose units of an orally dispersible granulate.

5. A process according to any one of preceding Claims 1 to 4, **characterised in that** said active ingredient is selected from non-steroidal anti-inflammatories.

6. A process according to Claim 5, **characterised in that** said active ingredient is selected from salicylic acid derivatives, pyrazolone derivatives, para-aminophenol derivatives, N-phenyl anthranylic acid derivatives and propionic acid derivatives.

7. A process according to Claim 5, **characterised in that** said active ingredient is selected from ibuprofen, naproxen, flurbiprofen, phenoprofen, ketoprofen, fenbufen, pirprofen, oxaprozine, indoprofen and tiaprofenic acid.

8. A process according to Claim 7, **characterised in that** said active ingredient is selected from ibuprofen, naproxen and flurbiprofen.

9. A process according to any of preceding Claims 1 to 8, **characterised in that** said hydrophilic carboxylate polymer is selected from carboxyalkylcellulose polymers, hemiesters of alkylcellulose dicarboxylic acids, and copolymers of an alkenyl carboxy acid with alkyl esters of an alkenyl carboxy acid.

10. A process according to Claim 9, **characterised in that** said hydrophilic carboxylate polymer is selected from hydroxypropylmethylcellulose phthalate and succinate.

11. A process according to any of the preceding Claims from 1 to 10, **characterised in that** said lipid compound is selected from fatty acids, esters of fatty acids with aliphatic alcohols, fatty alcohols, and triglycerides of fatty acids.

12. A process according to Claim 11, **characterised in that** said lipid compound is selected from aliphatic alcohols having from 12 to 18 carbon atoms.

13. A process according to any of preceding Claims 1 to 12, **characterised in that** said hydrophilic natural polymer having high molecular weight is selected from guar gum, arabic gum, karaya gum, gellan gum, carrageenan, chitosan, galactane, Polglumyt^{™} and mixtures thereof.

14. A process according to Claim 13, **characterised in that** said hydrophilic natural polymer having high molecular weight is used as a mixture with microcrystalline cellulose.

15. An orally dispersible solid pharmaceutical form **characterised in that** it comprises an active ingredient coated with at least one hydrophilic carboxylate polymer and at least one lipid compound, said coated active ingredient being embedded in a matrix comprising at least one hydrophilic natural polymer having high molecular weight

16. An orally dispersible solid pharmaceutical form according to Claim 15, **characterised in that** said solid pharmaceutical form is selected from the group comprising tablets and granulates.

17. An orally dispersible solid pharmaceutical form according to any of preceding Claims 15 or 16, **characterised in that** said active ingredient is selected from non-steroidal anti-inflammatories.

18. An orally dispersible solid pharmaceutical form according to Claim 17, **characterised in that** said active ingredient is selected from the salicylic acid derivatives pyrazolone derivatives, para-aminophenol derivatives, N-phenyl anthranylic acid derivatives and propionic acid derivatives.

19. An orally dispersible solid pharmaceutical form according to Claim 18, **characterised in that** said active ingredient is selected from the ibuprofen, naproxen, flurbiprofen, fenoprofen, ketoprofen, fenbufen, pirprofen, oxaprozine, indoprofen and tiaprofenic acid.

20. An orally dispersible solid pharmaceutical form according to Claim 19, **characterised in that** said active ingredient is selected from ibuprofen, naproxen and flurbuprofen.

21. An orally dispersible solid pharmaceutical form according to any of preceding Claims 16 to 20, **characterised in that** said hydrophilic carboxylate polymer is selected from carboxyalkylcellulose polymers, carboxymethylcellulose and carboxypropylcellulose, hemiesters of dicarboxylic acids with alkyl cellulose, hydroxypropylmethylcellulose phthalate, hydroxypropylmethylcellulose succinate and cellulose acetophthalate, and copolymers of an alkenyl carboxy acid with alkyl esters of an alkenyl carboxy acid, copolymers of acrylic and methacrylic acid and/or acrylates and/or methacrylates.

22. Anorally dispersible solid pharmaceutical form according to Claim 21, **characterised in that** said hydrophilic carboxylate polymer is selected from hydroxypropylmethylcellulose phthalates containing from 5 to 10% molar of hydroxypropyl residues, from 18 to 24% molar of methoxy residues and from 21 to 35% molar of phthalyl residues.

23. An orally dispersible solid pharmaceutical form according to Claim 21, **characterised in that** said hydrophilic carboxylate polymer is selected from hemiesters of dicarboxylic acids with alkyl cellulose.

24. An orally dispersible solid pharmaceutical form according to Claim 23, **characterised in that** said hydrophilic carboxylate polymer is selected from hydroxypropylmethylcellulose phthalate and succinate.

25. An orally dispersible solid pharmaceutical form according to any of preceding Claims 15 to 24, **characterised in that** it comprises, for each part by weight of said active ingredient, from 0.67 to 0.001 parts by weight of said hydrophilic carboxylate polymer.

26. An orally dispersible solid pharmaceutical form according to Claim 25, **characterised in that** it comprises, for each part by weight of said active ingredient, from 0.33 to 0.01 parts by weight of said hydrophilic carboxylate polymer.

27. An orally dispersible solid pharmaceutical form according to Claim 26, **characterised in that** it comprises, for each part by weight of said active ingredient, from 0.175 to 0.05 parts by weight of said hydrophilic carboxylate polymer.

28. An orally dispersible solid pharmaceutical form according to any of preceding Claims 15 to 27, **characterised in that** said lipid compound is selected from fatty acids, fatty acid esters with aliphatic alcohols, fatty alcohols and triglycerides of fatty acids.

29. An orally dispersible solid pharmaceutical form according to Claim 28, **characterised in that** said lipid compound is selected from aliphatic alcohols having 12 to 18 carbon atoms.

30. An orally dispersible solid pharmaceutical form according to any of preceding Claims 15 to 29, **characterised in that** it comprises, for each part by weight of said active ingredient, from 0.33 to 0.001 parts of said lipid compound.

31. An orally dispersible solid pharmaceutical form according to Claim 30, **characterised in that** it comprises, for each part by weight of said active ingredient, parts 0.25 to 0.01 of said lipid compound.

32. An orally dispersible solid pharmaceutical form according to Claim 31, **characterised in that** it comprises, for each part by weight of said active ingredient, 0.175 to 0.05 parts of said lipid compound.

33. An orally dispersible solid pharmaceutical form according to any of preceding Claims 15 to 32, **characterised in that** said hydrophilic natural polymer having high molecular weight is selected from guar gum, arabic gum, karaya gum, gellam gum, carrageenan, chitosan, galactan, Polglumyt^{™} and mixtures thereof.

34. An orally dispersible solid pharmaceutical form according to any of preceding Claims 15 to 33, **characterised in that** it comprises, for each part by weight of said active ingredient, from 0.33 to 0.001 parts of said hydrophilic natural polymer having high molecular weight

35. An orally dispersible solid pharmaceutical form according to Claim 34, **characterised in that** it comprises, for each part by weight of said active ingredient, from 0.25 to 0.005 parts of said hydrophilic natural polymer having high molecular weight.

36. An orally dispersible solid pharmaceutical form according to Claim 34, **characterised in that** it comprises, for each part by weight of said active ingredient, from 0.175 to 0.01 parts of said hydrophilic natural polymer having high molecular weight.

37. An orally dispersible solid pharmaceutical form according to any of preceding Claims 15 to 36, **characterised in that** said hydrophilic natural polymer having high molecular weight is used in a mixture with microcrystalline cellulose.

38. An orally dispersible solid pharmaceutical form according to Claim 37, **characterised in that** said mixture comprises from 4 to 10 parts by weight of microcrystalline cellulose for each part by weight of guar gum.

39. An orally dispersible solid pharmaceutical form according to any of preceding Claims 37 and 38, **characterised in that** it comprises, for each part by weight of said active ingredient, from 1.2 to 0.1 parts of said mixture of hydrophilic natural polymer having high molecular weight with microcrystalline cellulose.

40. An orally dispersible solid pharmaceutical form according to Claim 39, **characterised in that** it comprises, for each part by weight of said active ingredient, 1.0 to 0.2 parts of said mixture of hydrophilic natural polymer having high molecular weight with microcrystalline cellulose.

41. An orally dispersible solid pharmaceutical form according to any of preceding Claims 15 to 40, **characterised in that** the release of active ingredient, when measured in phosphate buffer at pH 7.2 by means of HPLC, is equal to or higher than 58% after five minutes.

42. An orally dispersible solid pharmaceutical form according to any of preceding Claims 15 to 41, **characterised in that** the releasing profile for the active ingredient, when measured in phosphate buffer at pH 7.2 by means of HPLC, shows the following trend:
| Time (min) | 5 min | 10 min | 15 min | 20 min | 45 min |
|---|---|---|---|---|---|
| Release (%) | >60% | >75% | >80% | >85% | >90% |

43. An orally dispersible solid pharmaceutical form according to any of preceding Claims 15 to 42, **characterised in that** the releasing profile for the active ingredient, when measured in phosphate buffer at pH 7.2 by means of HPLC, shows the following trend:
| Time (min) | 5 min | 10 min | 15 min | 20 min | 45 min |
|---|---|---|---|---|---|
| Release (%) | 60%-80% | 75%-85% | 80%-90% | 85%-95% | 90%-100% |

## Patentansprüche

1. Verfahren zur Herstellung einer oral dispergierbaren festen pharmazeutischen Form, **dadurch gekennzeichnet, dass** es die folgenden Schritte aufweist:
a) Beschichten des Wirkstoffs mit wenigstens einem hydrophilen Carboxylatpolymer,
b) Granulieren des in Schritt a) erhaltenen beschichteten Wirkstoffs mit wenigstens einer Lipidverbindung, deren Schmelzpunkt niedriger als der des Wirkstoffs ist,
c) Mischen des in Schritt b) erhaltenen Granulats mit wenigstens einem hochmolekularen hydrophilen natürlichen Polymer, und
d) Mischen des in Schritt c) erhaltenen Granulats mit geeigneten Bestandteilen zum Erhalt einer oral dispergierbaren festen pharmazeutischen Form.

2. Verfahren nach Anspruch 1, **dadurch gekennzeichnet, dass** die feste pharmazeutische Form unter Tabletten und Granulaten ausgewählt ist.

3. Verfahren nach Anspruch 1, **dadurch gekennzeichnet, dass** es nach dem Schritt d) den Schritt aufweist:
e) Komprimieren der in Schritt d) erhaltenen Formulierung, wobei man oral dispergierbare Tabletten erhält.

4. Verfahren nach Anspruch 1, **dadurch gekennzeichnet, dass** es nach dem Schritt d) den Schritt aufweist:
e) Aufteilen der in Schritt d) erhaltenen Formulierung, wobei man Dosiseinheiten eines oral dispergierbaren Granulats erhält.

5. Verfahren nach einem der vorhergehenden Ansprüche 1 bis 4, **dadurch gekennzeichnet, dass** der Wirkstoff unter nicht-steroidalen Entzündungshemmern ausgewählt ist.

6. Verfahren nach Anspruch 5, **dadurch gekennzeichnet, dass** der Wirkstoff unter Salicylsäurederivaten, Pyrazolonderivaten, para-Aminophenolderivaten, N-Phenylanthranilsäurederivaten und Propionsäurederivaten ausgewählt ist.

7. Verfahren nach Anspruch 5, **dadurch gekennzeichnet, dass** der Wirkstoff unter Ibuprofen, Naproxen, Flurbiprofen, Fenoprofen, Ketoprofen, Fenbufen, Pirprofen, Oxaprozin, Indoprofen und Tiaprofensäure ausgewählt ist.

8. Verfahren nach Anspruch 7, **dadurch gekennzeichnet, dass** der Wirkstoff unter Ibuprofen, Naproxen und Flurbiprofen ausgewählt ist.

9. Verfahren nach einem der vorhergehenden Ansprüche 1 bis 8, **dadurch gekennzeichnet, dass** das hydrophile Carboxylatpolymer unter Carboxyalkylcellulosepolymeren, Halbestern von Alkylcellulosedicarbonsäuren und Copolymeren einer Alkenylcarbonsäure mit Alkylestern einer Alkenylcarbonsäure ausgewählt ist.

10. Verfahren nach Anspruch 9, **dadurch gekennzeichnet, dass** das hydrophile Carboxylatpolymer unter Hydroxypropylmethylcellulosephthalat und -succinat ausgewählt ist.

11. Verfahren nach einem der vorhergehenden Ansprüche 1 bis 10, **dadurch gekennzeichnet, dass** die Lipidverbindung unter Fettsäuren, Estern von Fettsäuren mit aliphatischen Alkoholen, Fettalkoholen und Fettsäuretriglyceriden ausgewählt ist.

12. Verfahren nach Anspruch 11, **dadurch gekennzeichnet, dass** die Lipidverbindung unter aliphatischen Alkoholen mit 12 bis 18 Kohlenstoffatomen ausgewählt ist.

13. Verfahren nach einem der vorhergehenden Ansprüche 1 bis 12, **dadurch gekennzeichnet, dass** das hochmolekulare hydrophile natürliche Polymer unter Guargummi, Gummi arabicum, Karayagummi, Gellangummi, Carrageenan, Chitosan, Galactan, Polglumyt^{®} und Gemischen davon ausgewählt ist.

14. Verfahren nach Anspruch 13, **dadurch gekennzeichnet, dass** das hochmolekulare hydrophile natürliche Polymer als Gemisch mit mikrokristalliner Cellulose eingesetzt wird.

15. Oral dispergierbare feste pharmazeutische Form, **dadurch gekennzeichnet, dass** sie einen mit wenigstens einem hydrophilen Carboxylatpolymer beschichteten Wirkstoff und wenigstens eine Lipidverbindung umfasst, wobei der beschichtete Wirkstoff in einer Matrix eingebettet ist, die wenigstens ein hochmolekulares hydrophiles natürliches Polymer umfaßt.

16. Oral dispergierbare feste pharmazeutische Form nach Anspruch 15, **dadurch gekennzeichnet, dass** die feste pharmazeutische Form unter Tabletten und Granulaten ausgewählt ist.

17. Oral dispergierbare feste pharmazeutische Form nach einem der vorhergehenden Ansprüche 15 oder 16, **dadurch gekennzeichnet, dass** der Wirkstoff unter nicht-steroidalen Entzündungshemmern ausgewählt ist.

18. Oral dispergierbare feste pharmazeutische Form nach Anspruch 17, **dadurch gekennzeichnet, dass** der Wirkstoff unter Salicylsäurederivaten, Pyrazolonderivaten, para-Aminophenolderivaten, N-Phenylanthranilsäurederivaten und Propionsäurederivaten ausgewählt ist.

19. Oral dispergierbare feste pharmazeutische Form nach Anspruch 18, **dadurch gekennzeichnet, dass** der Wirkstoff unter Ibuprofen, Naproxen, Flurbiprofen, Fenoprofen, Ketoprofen, Fenbufen, Pirprofen, Oxaprozin, Indoprofen and Tiaprofensäure ausgewählt ist.

20. Oral dispergierbare feste pharmazeutische Form nach Anspruch 19, **dadurch gekennzeichnet, dass** der Wirkstoff unter Ibuprofen, Naproxen und Flurbiprofen ausgewählt ist.

21. Oral dispergierbare feste pharmazeutische Form nach einem der vorhergehenden Ansprüche 16 bis 20, **dadurch gekennzeichnet, dass** das hydrophile Carboxylatpolymer unter Carboxyalkylcellulosepolymeren, Carboxymethylcellulose und Carboxypropylcellulose, Halbestern von Dicarbonsäuren mit Alkylcellulose, Hydroxypropylmethylcellulosephthalat, Hydroxypropylmethylcellulosesuccinat und Celluloseacetophthalat, und Copolymeren einer Alkenylcarbonsäure mit Alkylestern einer Alkenylcarbonsäure, Copolymeren von Acryl- und Methacrylsäure und/oder Acrylaten und/oder Methacrylaten ausgewählt ist.

22. Oral dispergierbare feste pharmazeutische Form nach Anspruch 21, **dadurch gekennzeichnet, dass** das hydrophile Carboxylatpolymer unter Hydroxypropylmethylcellulosephthalaten ausgewählt ist, die 5 - 10 Mol-% Hydroxypropylreste, 18 - 24 Mol-% Methoxyreste und 21 - 35 Mol-% Phthalylreste enthalten.

23. Oral dispergierbare feste pharmazeutische Form nach Anspruch 21, **dadurch gekennzeichnet, dass** das hydrophile Carboxylatpolymer unter Halbestern von Dicarbonsäuren mit Alkylcellulose ausgewählt ist.

24. Oral dispergierbare feste pharmazeutische Form nach Anspruch 23, **dadurch gekennzeichnet, dass** das hydrophile Carboxylatpolymer unter Hydroxypropylmethylcellulosephthalat und -succinat ausgewählt ist.

25. Oral dispergierbare feste pharmazeutische Form nach einem der vorhergehenden Ansprüche 15 bis 24, **dadurch gekennzeichnet, dass** sie auf einen Gewichtsteil Wirkstoff 0,67 bis 0,001 Gewichtsteile des hydrophilen Carboxylatpolymers umfasst.

26. Oral dispergierbare feste pharmazeutische Form nach Anspruch 25, **dadurch gekennzeichnet, dass** sie auf einen Gewichtsteil Wirkstoff 0,33 bis 0,01 Gewichtsteile des hydrophilen Carboxylatpolymers umfasst.

27. Oral dispergierbare feste pharmazeutische Form nach Anspruch 26, **dadurch gekennzeichnet, dass** sie auf einen Gewichtsteil Wirkstoff 0,175 bis 0,05 Gewichtsteile des hydrophilen Carboxylatpolymers umfasst.

28. Oral dispergierbare feste pharmazeutische Form nach einem der vorhergehenden Ansprüche 15 bis 27, **dadurch gekennzeichnet, dass** die Lipidverbindung unter Fettsäuren, Fettsäureestern mit aliphatischen Alkoholen, Fettalkoholen und Fettsäuretrigliceriden ausgewählt ist.

29. Oral dispergierbare feste pharmazeutische Form nach Anspruch 28, **dadurch gekennzeichnet, dass** die Lipidverbindung unter aliphatischen Alkoholen mit 12 bis 18 Kohlenstoffatomen ausgewählt ist.

30. Oral dispergierbare feste pharmazeutische Form nach einem der vorhergehenden Ansprüche 15 bis 29, **dadurch gekennzeichnet, dass** sie auf einen Gewichtsteil Wirkstoff 0,33 bis 0,001 Teile der Lipidverbindung umfasst.

31. Oral dispergierbare feste pharmazeutische Form nach Anspruch 30, **dadurch gekennzeichnet, dass** sie auf einen Teil Wirkstoff 0,25 bis 0,01 Teile der Lipidverbindung umfasst.

32. Oral dispergierbare feste pharmazeutische Form nach Anspruch 31, **dadurch gekennzeichnet, dass** sie auf einen Gewichtsteil Wirkstoff 0,175 bis 0,05 Teile der Lipidverbindung umfasst.

33. Oral dispergierbare feste pharmazeutische Form nach einem der vorhergehenden Ansprüche 15 bis 32, **dadurch gekennzeichnet, dass** das hochmolekulare hydrophile natürliche Polymer unter Guargummi, Gummi arabicum, Karayagummi, Gellangummi, Carrageenan, Chitosan, Galactan, Polglumyt^{®} und Gemischen davon ausgewählt ist.

34. Oral dispergierbare feste pharmazeutische Form nach einem der vorhergehenden Ansprüche 15 bis 33, **dadurch gekennzeichnet, dass** sie auf einen Gewichtsteil Wirkstoff 0,33 bis 0,001 Teile des hochmolekularen hydrophilen natürlichen Polymers umfasst.

35. Oral dispergierbare feste pharmazeutische Form nach Anspruch 34, **dadurch gekennzeichnet, dass** sie auf einen Gewichtsteil Wirkstoff 0,25 bis 0,005 Teile des hochmolekularen hydrophilen natürlichen Polymers umfasst.

36. Oral dispergierbare feste pharmazeutische Form nach Anspruch 34, **dadurch gekennzeichnet, dass** sie auf einen Gewichtsteil Wirkstoff 0,175 bis 0,01 Teile des hochmolekularen hydrophilen natürlichen Polymers umfasst.

37. Oral dispergierbare feste pharmazeutische Form nach einem der vorhergehenden Ansprüche 15 bis 36, **dadurch gekennzeichnet, dass** das hochmolekulare hydrophile natürliche Polymer im Gemisch mit mikrokristalliner Cellulose verwendet wird.

38. Oral dispergierbare feste pharmazeutische Form nach Anspruch 37, **dadurch gekennzeichnet, dass** das Gemisch 4 bis 10 Gewichtsteile mikrokristalline Cellulose auf einen Gewichtsteil Guargummi umfasst.

39. Oral dispergierbare feste pharmazeutische Form nach einem der vorhergehenden Ansprüche 37 und 38, **dadurch gekennzeichnet, dass** sie auf einen Gewichtsteil Wirkstoff 1,2 bis 0,1 Teile des Gemisches des hochmolekularen hydrophilen natürlichen Polymers mit mikrokristalliner Cellulose umfasst.

40. Oral dispergierbare feste pharmazeutische Form nach Anspruch 39, **dadurch gekennzeichnet, dass** sie auf einen Gewichtsteil Wirkstoff 1,0 bis 0,2 Teile des Gemisches des hochmolekularen hydrophilen natürlichen Polymers mit mikrokristalliner Cellulose umfasst.

41. Oral dispergierbare feste pharmazeutische Form nach einem der vorhergehenden Ansprüche 15 bis 40, **dadurch gekennzeichnet, dass** die Freisetzung des Wirkstoffs, bei Bestimmung in Phosphatpuffer bei pH 7,2 durch HPLC, nach 5 Minuten 58% oder mehr beträgt.

42. Oral dispergierbare feste pharmazeutische Form nach einem der vorhergehenden Ansprüche 15 bis 41, **dadurch gekennzeichnet, dass** das Freisetzungsprofil für den Wirkstoff, bei Bestimmung in Phosphatpuffer bei pH 7,2 durch HPLC, den folgenden Verlauf zeigt:
| Zeit (min) | 5 min | 10 min | 15 min | 20 min | 45 min |
|---|---|---|---|---|---|
| Freisetzung (%) | >60% | >75% | >80% | >85% | >90% |

43. Oral dispergierbare feste pharmazeutische Form nach einem der vorhergehenden Ansprüche 15 bis 42, **dadurch gekennzeichnet, dass** das Freisetzungsprofil für den Wirkstoff, bei Bestimmung in Phosphatpuffer bei pH 7,2 durch HPLC, den folgenden Verlauf zeigt:
| Zeit (min) | 5 min | 10 min | 15 min | 20 min | 45 min |
|---|---|---|---|---|---|
| Freisetzung (%) | 60% - 80% | 75% -85% | 80% - 90% | 85% - 95% | 90%-100% |

## Revendications

1. Procédé de préparation d'une forme pharmaceutique solide orodispersible, **caractérisé en ce qu'**il comprend les étapes suivantes:
a. l'enrobage de l'ingrédient actif avec au moins un polymère carboxylate hydrophile,
b. la granulation de l'ingrédient actif enrobé obtenu dans l'étape (a) avec au moins un composé lipidique ayant un point de fusion inférieur à celui de l'ingrédient actif,
c. le mélange du granulé obtenu dans l'étape (b) avec au moins un polymère naturel hydrophile ayant une masse molaire élevée, et
d. le mélange du granulé obtenu dans l'étape (c) avec des ingrédients appropriés pour l'obtention d'une forme pharmaceutique solide orodispersible.

2. Procédé selon la revendication 1, **caractérisé en ce que** ladite forme pharmaceutique solide est choisie parmi des comprimés et des granulés.

3. Procédé selon la revendication 1, **caractérisé en ce que,** après l'étape (d), il comprend l'étape de
(e) compression de la formulation obtenue dans l'étape (d) pour l'obtention de comprimés orodispersibles.

4. Procédé selon la revendication 1, **caractérisé en ce que,** après l'étape (d), il comprend l'étape de
(e) subdivision de la formulation obtenue dans l'étape (d) pour l'obtention d'unités de prise d'un granulé orodispersible.

5. Procédé selon l'une quelconque des revendications 1 à 4 précédentes, **caractérisé en ce que** ledit ingrédient actif est choisi parmi des anti-inflammatoires non stéroïdiens.

6. Procédé selon la revendication 5, **caractérisé en ce que** ledit ingrédient actif est choisi parmi des dérivés de l'acide salicylique, des dérivés de pyrazolone, des dérivés de para-aminophénol, des dérivés de l'acide N-phénylanthranylique et des dérivés de l'acide propionique.

7. Procédé selon la revendication 5, **caractérisé en ce que** ledit ingrédient actif est choisi parmi l'ibuprofène, le naproxène, le flurbiprofène, le phénoprofène, le kétoprofène, le fenbufène, le pirprofène, l'oxaprozine, l'indoprofène et l'acide tiaprofénique.

8. Procédé selon la revendication 7, **caractérisé en ce que** ledit ingrédient actif est choisi parmi l'ibuprofène, le naproxène et le flurbiprofène.

9. Procédé selon l'une quelconque des revendications 1 à 8 précédentes, **caractérisé en ce que** ledit polymère carboxylate hydrophile est choisi parmi les polymères carboxyalkylcellulose, les hémiesters d'acides dicarboxyliques avec une alkylcellulose, et les copolymères d'un acide alcénylcarboxylique avec des esters alkyliques d'un acide alcénylcarboxylique.

10. Procédé selon la revendication 9, **caractérisé en ce que** ledit polymère carboxylate hydrophile est choisi parmi un phtalate et un succinate d'hydroxypropyl-méthylcellulose.

11. Procédé selon l'une quelconque des revendications 1 à 10 précédentes, **caractérisé en ce que** ledit composé lipidique est choisi parmi les acides gras, les esters d'acides gras avec des alcools aliphatiques, les alcools gras, et les triglycérides d'acides gras.

12. Procédé selon la revendication 11, **caractérisé en ce que** ledit composé lipidique est choisi parmi les alcools aliphatiques de 12 à 18 atomes de carbone.

13. Procédé selon l'une quelconque des revendications 1 à 12 précédentes, **caractérisé en ce que** ledit polymère naturel hydrophile ayant une masse molaire élevée est choisi parmi la gomme de guar, la gomme arabique, la gomme karaya, le gomme de gellane, la carragénine, le chitosane, le galactane, le Polglumyt^{®} et leurs mélanges.

14. Procédé selon la revendication 13, **caractérisé en ce que** ledit polymère naturel hydrophile ayant une masse molaire élevée est utilisé sous forme de mélange avec de la cellulose microcristalline.

15. Forme pharmaceutique solide orodispersible, **caractérisée en ce qu'**elle comprend un ingrédient actif enrobé avec au moins un polymère carboxylate hydrophile et au moins un composé lipidique, ledit ingrédient actif enrobé étant inclus dans une matrice comprenant au moins un polymère naturel hydrophile ayant une masse molaire élevée.

16. Forme pharmaceutique solide orodispersible selon la revendication 15, **caractérisée en ce que** ladite forme pharmaceutique solide est choisie parmi des comprimés et des granulés.

17. Forme pharmaceutique solide orodispersible selon l'une quelconque des revendications 15 ou 16, **caractérisée en ce que** ledit ingrédient actif est choisi parmi des anti-inflammatoires non stéroïdiens.

18. Forme pharmaceutique solide orodispersible selon la revendication 17, **caractérisée en ce que** ledit ingrédient actif est choisi parmi des dérivés de l'acide salicylique, des dérivés de pyrazolone, des dérivés de para-aminophénol, des dérivés de l'acide N-phénylanthranylique et des dérivés de l'acide propionique.

19. Forme pharmaceutique solide orodispersible selon la revendication 18, **caractérisée en ce que** ledit ingrédient actif est choisi parmi l'ibuprofène, le naproxène, le flurbiprofène, le fénoprofène, le kétoprofène, le fenbufène, le pirprofène, l'oxaprozine, l'indoprofène et l'acide tiaprofénique.

20. Forme pharmaceutique solide orodispersible selon la revendication 19, **caractérisée en ce que** ledit ingrédient actif est choisi parmi l'ibuprofène, le naproxène et le flurbiprofène.

21. Forme pharmaceutique solide orodispersible selon l'une quelconque des revendications 16 à 20 précédentes, **caractérisée en ce que** ledit polymère carboxylate hydrophile est choisi parmi les polymères carboxyalkylcellulose, une carboxyméthylcellulose et une carboxypropylcellulose, les hémiesters d'acides dicarboxyliques avec une alkylcellulose, un phtalate d'hydroxypropylméthylcellulose, un succinate d'hydroxypropylméthylcellulose et des copolymères d'un acide alcénylcarboxylique avec des esters alkyliques d'un acide alcénylcarboxylique, des copolymères d'acide acrylique et méthacrylique et/ou d'acrylates et/ou de méthacrylates.

22. Forme pharmaceutique solide orodispersible selon la revendication 21, **caractérisée en ce que** le polymère carboxylate hydrophile est choisi parmi des phtalates d'hydroxypropylméthylcellulose contenant de 5 à 10 % en mol de résidus hydroxypropyle, de 18 à 24 % en mol de résidus méthoxy et de 21 à 35 % en mol de résidus phtalyle.

23. Forme pharmaceutique solide orodispersible selon la revendication 21, **caractérisée en ce que** ledit polymère carboxylate hydrophile est choisi parmi les hémiesters d'acides dicarboxyliques avec une alkylcellulose.

24. Forme pharmaceutique solide orodispersible selon la revendication 23, **caractérisée en ce que** ledit polymère carboxylate hydrophile est choisi parmi un phtalate et un succinate d'hydroxypropylméthylcellulose.

25. Forme pharmaceutique solide orodispersible selon l'une quelconque des revendications 15 à 24 précédentes, **caractérisée en ce qu'**elle comprend, pour chaque partie en masse dudit ingrédient actif, de 0,67 à 0,001 partie en masse dudit polymère carboxylate hydrophile.

26. Forme pharmaceutique solide orodispersible selon la revendication 25, **caractérisée en ce qu'**elle comprend, pour chaque partie en masse dudit ingrédient actif, de 0,33 à 0,01 partie en masse dudit polymère carboxylate hydrophile.

27. Forme pharmaceutique solide orodispersible selon la revendication 26, **caractérisée en ce qu'**elle comprend, pour chaque partie en masse dudit ingrédient actif, de 0,175 à 0,05 partie en masse dudit polymère carboxylate hydrophile.

28. Forme pharmaceutique solide orodispersible selon l'une quelconque des revendications 15 à 27 précédentes, **caractérisée en ce que** ledit composé lipidique est choisi parmi les acides gras, les esters d'acides gras avec des alcools aliphatiques, les alcools gras, et les triglycérides d'acides gras.

29. Forme pharmaceutique solide orodispersible selon la revendication 28, **caractérisée en ce que** ledit composé lipidique est choisi parmi les alcools aliphatiques de 12 à 18 atomes de carbone.

30. Forme pharmaceutique solide orodispersible selon l'une quelconque des revendications 15 à 29 précédentes, **caractérisée en ce qu'**elle comprend, pour chaque partie en masse dudit ingrédient actif, de 0,33 à 0,001 partie en masse dudit composé lipidique.

31. Forme pharmaceutique solide orodispersible selon la revendication 30, **caractérisée en ce qu'**elle comprend, pour chaque partie en masse dudit ingrédient actif, de 0,25 à 0,01 partie en masse dudit composé lipidique.

32. Forme pharmaceutique solide orodispersible selon la revendication 31, **caractérisée en ce qu'**elle comprend, pour chaque partie en masse dudit ingrédient actif, de 0,175 à 0,05 partie en masse dudit composé lipidique.

33. Forme pharmaceutique solide orodispersible selon l'une quelconque des revendications 15 à 32 précédentes, **caractérisée en ce que** ledit polymère naturel hydrophile ayant une masse molaire élevée est choisi parmi la gomme de guar, la gomme arabique, la gomme karaya, le gomme de gellane, la carragénine, le chitosane, le galactane, le Polglumyt^{®} et leurs mélanges.

34. Forme pharmaceutique solide orodispersible selon l'une quelconque des revendications 15 à 33 précédentes, **caractérisée en ce qu'**elle comprend, pour chaque partie en masse dudit ingrédient actif, de 0,33 à 0,001 partie en masse dudit polymère naturel hydrophile ayant une masse molaire élevée.

35. Forme pharmaceutique solide orodispersible selon la revendication 34, **caractérisée en ce qu'**elle comprend, pour chaque partie en masse dudit ingrédient actif, de 0,25 à 0,005 partie en masse dudit polymère naturel hydrophile ayant une masse molaire élevée.

36. Forme pharmaceutique solide orodispersible selon la revendication 34, **caractérisée en ce qu'**elle comprend, pour chaque partie en masse dudit ingrédient actif, de 0,175 à 0,01 partie en masse dudit polymère naturel hydrophile ayant une masse molaire élevée.

37. Forme pharmaceutique solide orodispersible selon l'une quelconque des revendications 15 à 36, **caractérisée en ce que** ledit polymère naturel hydrophile ayant une masse molaire élevée est utilisé en un mélange avec de la cellulose microcristalline.

38. Forme pharmaceutique solide orodispersible selon la revendication 37, **caractérisée en ce que** ledit mélange comprend de 4 à 10 parties en masse de cellulose microcristalline pour chaque partie en masse de gomme de guar.

39. Forme pharmaceutique solide orodispersible selon l'une quelconque des revendications 37 et 38 précédentes, **caractérisée en ce qu'**elle comprend, pour chaque partie en masse dudit ingrédient actif, de 1,2 à 0,1 partie en masse dudit mélange de polymère naturel hydrophile ayant une masse molaire élevée avec de la cellulose microcristalline.

40. Forme pharmaceutique solide orodispersible selon la revendication 39, **caractérisée en ce qu'**elle comprend, pour chaque partie en masse dudit ingrédient actif, de 1,0 à 0,2 partie en masse dudit mélange de polymère naturel hydrophile ayant une masse molaire élevée avec de la cellulose microcristalline.

41. Forme pharmaceutique solide orodispersible selon l'une quelconque des revendications 15 à 40 précédentes, **caractérisée en ce que** la libération de l'ingrédient actif, mesurée dans du tampon phosphate à pH 7,2 par CLHP, est égale ou supérieure à 58 % au bout de 5 minutes.

42. Forme pharmaceutique solide orodispersible selon l'une quelconque des revendications 15 à 41 précédentes, **caractérisée en ce que** le profil de libération de l'ingrédient actif, mesuré dans du tampon phosphate à pH 7,2 par CLHP, présente la tendance suivante:
| temps (min) | 5 min | 10 min | 15 min | 20 min | 45 min |
|---|---|---|---|---|---|
| libération (%) | >60% | >75% | >80% | >85% | >90% |

43. Forme pharmaceutique solide orodispersible selon l'une quelconque des revendications 15 à 42 précédentes, **caractérisée en ce que** le profil de libération de l'ingrédient actif, mesuré dans du tampon phosphate à pH 7,2 par CLHP, présente la tendance suivante:
| temps (min) | 5 min | 10 min | 15 min | 20 min | 45 min |
|---|---|---|---|---|---|
| libération (%) | 60-80 % | 75-85 % | 80-90 % | 85-95 % | 90-100 % |
